(19)

Europäisches
Patentamt
European
Patent Office
Office européen
des brevets

(11) **EP 4 197 471 A1**

(12) **EUROPEAN PATENT APPLICATION**

(43) Date of publication:
**21.06.2023 Bulletin 2023/25**

(21) Application number: **22213304.3**

(22) Date of filing: **14.12.2022**

(51) International Patent Classification (IPC):
**A61B 18/14** (2006.01)      **A61B 18/00** (2006.01)

(52) Cooperative Patent Classification (CPC):
**A61B 18/1492;** A61B 2018/0022;
A61B 2018/00327; A61B 2018/00375;
A61B 2018/00541; A61B 2018/00577;
A61B 2018/00875

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.12.2021 US 202117551952**

(71) Applicant: **Biosense Webster (Israel) Ltd
2066717 Yokneam (IL)**

(72) Inventors:
• **ADAWI, Eid
Yokneam 2066717 (IL)**
• **GOVARI, Assaf
Yokneam 2066717 (IL)**

(74) Representative: **Carpmaels & Ransford LLP
One Southampton Row
London WC1B 5HA (GB)**

(54) **BALLOON ABLATION CATHETER IMPEDANCE MEASUREMENT FOR LESION ASSESSMENT**

(57)      A system for use with performing a medical procedure is provided which comprises a balloon catheter and a processing device. The balloon catheter comprises a plurality of ablation electrodes configured to ablate tissue of patient anatomy, a stem electrode and an edge electrode. The processing device comprises a processor configured to acquire first impedance measurements between each of the ablation electrodes of the balloon catheter and an edge electrode of the balloon catheter, acquire second impedance measurements between each of the ablation electrodes of the balloon catheter and a stem electrode of the balloon catheter, determine, during ablation of the tissue, changes to at least one of the first and second impedance measurements, and indicating lesion formation based on the changes to at least one of the first and second impedance measurements.

EP 4 197 471 A1

**Description**

FIELD OF INVENTION

The present application relates the field of medical diagnosis and treatment using medical probes and in particular, to assessment of lesion formation during ablation using multi-electrode cardiac ablation catheters.

BACKGROUND

Medical personnel, such as ear, nose and throat (ENT) physicians and cardiologists, use medical tools for performing medical procedures within patient anatomy. Medical tools, such as multi-electrode cardiac ablation catheters, are used to ablate portions of dysfunctional tissue, such as tissue of a heart, lung, ear, nose, throat or other organs. For example, a radio-frequency (RF) catheter ablation procedure typically includes inserting a catheter through an incision in the skin and guiding the catheter to an organ where the catheter is used to create ablation lesions on the organ tissue.

SUMMARY

A method of lesion assessment for a medical ablation procedure is provided which comprises acquiring first impedance measurements between each of a plurality of ablation electrodes of a medical probe and a stem electrode of the medical probe, acquiring second impedance measurements between each of the ablation electrodes of the medical probe and an edge electrode of the medical probe, ablating tissue of patient anatomy, determining, during ablation of the tissue, changes to at least one of the first and second impedance measurements, and indicating lesion formation based on the changes to at least one of the first and second impedance measurements.

A processing device for use during a medical procedure is provided which comprises memory configured to store data and a processor. The processor is configured to acquire first impedance measurements between each of a plurality of ablation electrodes of a medical probe and an edge electrode of the medical probe, acquire second impedance measurements between each of the ablation electrodes and a stem electrode of the medical probe, determine, during ablation of tissue of patient anatomy, changes to at least one of the first and second impedance measurements, and indicating lesion formation based on the changes to at least one of the first and second impedance measurements.

A system for use with performing a medical procedure is provided which comprises a balloon catheter and a processing device. The balloon catheter comprises a plurality of ablation electrodes configured to ablate tissue of patient anatomy, a stem electrode and an edge electrode. The processing device comprises a processor configured to acquire first impedance measurements between each of the ablation electrodes of the balloon catheter and an edge electrode of the balloon catheter, acquire second impedance measurements between each of the ablation electrodes of the balloon catheter and a stem electrode of the balloon catheter, determine, during ablation of the tissue, changes to at least one of the first and second impedance measurements, and indicating lesion formation based on the changes to at least one of the first and second impedance measurements.

BRIEF DESCRIPTION OF THE DRAWINGS

A more detailed understanding can be had from the following description, given by way of example in conjunction with the accompanying drawings wherein:

FIG. 1 an illustration of an example of a catheter-based position-tracking and ablation system comprising an ablation balloon catheter for implementing features of the present disclosure;
FIG. 2 is a block diagram illustrating example components of a medical system for use with embodiments described herein;
FIG. 3 is an illustration of the example catheter shown in FIG. 1 in physical contact with cavity wall tissue;
FIG. 4A is a schematic diagram of an ablation electrode electrically coupled to the edge electrode shown in FIG. 3 while the ablation electrode is in partial contact with the tissue;
FIG. 4B is a schematic diagram of an ablation electrode electrically coupled to the edge electrode shown in FIG. 3 while the ablation electrode is in full contact with the tissue;
FIG. 5 is a flow chart illustrating a method of lesion assessment formation according to features of the present disclosure;
FIG. 6 illustrates the display of different types of acquired impedance measurement information for use as lesion assessment according to features of the present disclosure; and
FIG. 7 is a flow chart illustrating an example method of determining a level of contact sufficiency and indicating changes between relative bipolar impedance values according to features of the present disclosure.

DETAILED DESCRIPTION

As used herein, the terms "about" or "approximately" for any numerical values or ranges indicate a suitable dimensional tolerance which allows the part or collection of components to function for its intended purpose as described herein. More specifically, "about" or "approximately" can refer to the range of values $\pm 20\%$ of the recited value. For example, "about 90%" can indicate a range of values from 71% to 99%.

Dynamic maps of the patient anatomy (e.g., organs) are created to facilitate accurate determination of regions for ablation. Target ablation sites (i.e., regions of interest (ROI)) of an organ are identified by viewing the maps. Based on the identified ablation sites, an ablation procedure, which includes one or more ablations, is performed on the organ.

A multi-electrode ablation catheter, such as a balloon ablation catheter or a basket catheter, can be used to perform the ablation procedure. A multi-electrode ablation catheter typically comprises an expandable frame (e.g., an inflatable balloon), which is coupled to the distal end of a shaft for insertion into a cavity of an organ of a patient, and a plurality of ablating electrodes disposed over the frame.

Successful ablation treatment is facilitated when each of the ablating electrodes are in physical contact with cavity wall tissue to be ablated. For example, when a balloon catheter with multiple ablation electrodes is used to ablate an ostium of a pulmonary ventricle (PV), typically each of the ablation electrodes of the catheter should be positioned so they are in full contact with the PV tissue. The ablation electrodes are often not in full contact with tissue, however, and portions of ablation electrodes are instead immersed in blood, resulting in these electrodes not ablating tissue and potentially causing problematic side effects such as clot formation.

Some conventional techniques determine if each of the electrodes of an ablation catheter are in full contact with tissue based on impedance measurements between ablation electrodes and a stem electrode as well as between the ablation electrodes and the edge electrode (i.e., tip electrode) on the catheter. While these conventional techniques provide a good indication of the ablation electrodes being in full contact with the tissue, these techniques do not provide an indication as to whether an ablation resulted in a lesion being formed.

Features of the present disclosure provide additional techniques for performing a lesion assessment by monitoring changes to impedance characteristics during ablation. The lesion assessment includes measurements (e.g., impedance changes), in addition to the impedance measurements for determining full contact with tissue, and providing (e.g., displaying) the results of the additional measurements physician for predicting whether or not a lesion was formed. The lesion assessment is performed by acquiring impedance measurements between ablation electrodes and both stem and edge electrodes before ablation, acquiring second impedance measurements between the ablation electrodes and both the stem and edge electrodes during ablation and determining changes between the first and second impedance measurements during ablation. A change in the impedance measurements is indicative of the formation of a lesion.

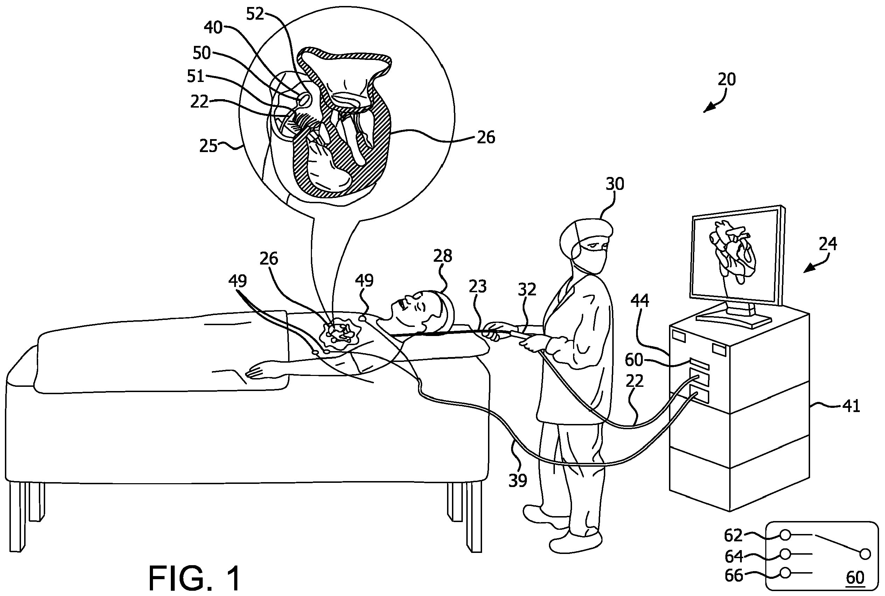

FIG. 1 an illustration of an example of a catheter-based position-tracking and ablation system 20 comprising an ablation balloon catheter 40 and computing device 24 for implementing features of the present disclosure. Typically, balloon catheter 40 is used for therapeutic treatment, such as ablating cardiac tissue, for example at the left atrium. System 20 is used to determine the position of balloon catheter 40, shown in inset 25, coupled to a distal end of a shaft 22. System 20 is further used to determine (e.g., prior to performing an ablation), whether each of ablation electrodes 50 of balloon catheter 40 is in contact with tissue.

The example computing device 24 shown in FIG. 1 includes a processor 41 and interface circuits 44 for transmitting and receiving signals, such as RF signals and position signals. Interface circuits 44 can also receive electrocardiograms from surface electrodes 49 and/or from any electrode disposed on the catheter 40.

In some embodiments, processor 41 controls a relay 60 to switch electrical connections between two or more of: (i) a first configuration having a connection (62) between the ablation electrodes and surface electrodes 49 for measuring impedances between the ablation electrodes and one or more body-surface electrodes, (ii) a second configuration having a connection (64) between the ablation electrodes and the stem and edge electrodes of the balloon catheter for measuring impedances between the ablation electrodes and the stem and edge electrodes, where connections 62 and 64 are used in order to interchangeably measure electrode position and degree of electrode contact with tissue at the location, and (iii) a connection (66) between the ablation electrodes and a back patch electrode (not shown) in order to perform ablation by driving electrical signal between the ablation electrodes and the back patch electrode.

Physician 30 navigates balloon catheter 40 to a target location in a heart 26 of a patient 28 by manipulating shaft 22 using a manipulator 32 near the proximal end of the catheter and/or deflection from a sheath 23. Balloon catheter 40 is inserted, in a folded configuration, through sheath 23, and only after the balloon is retracted from the sheath 23 does balloon catheter 40 regain its intended functional shape. By containing balloon catheter 40 in a folded configuration, sheath 23 also serves to minimize vascular trauma on its way to the target location.

Balloon catheter 40 comprises elongated and large area ablation electrodes 50, which are disposed on an outer surface of the balloon membrane. A stem electrode 51 is disposed on a distal end of shaft 22 proximally to the balloon. An edge electrode 52 is disposed on the distal end of shaft 22 just distally to the balloon. Electrodes 51 and 52 are used to determine whether each of ablation electrodes 50 is in full contact with tissue or at least partially immersed in blood.

Ablation electrodes 50, stem electrode 51, and edge electrode 52 are electrically connected to each other, for example, via wires running through shaft 22 to interface circuits 44 in computing device 24. Additionally, ablation electrodes 50 can be used to determine a position of balloon catheter 40 inside heart 26, by sensing impedances relative to surface electrodes 49 via wires running through a cable 39 to the chest of patient 28. The position of the balloon catheter 40 is determined, for example, using advanced catheter location (ACL), which uses a position tracking subsystem that measures impedances between the ablation electrodes and surface electrodes to track positions of electrodes on the balloon catheter inside the organ. ACL is implemented in various medical applications, for example in the CARTO system, produced by Biosense-Webster Inc. (Irvine, California) and described in U.S. Patents 7,756,576, 7,869,865, 7,848,787, and 8,456,182, whose disclosures are all incorporated herein by reference.

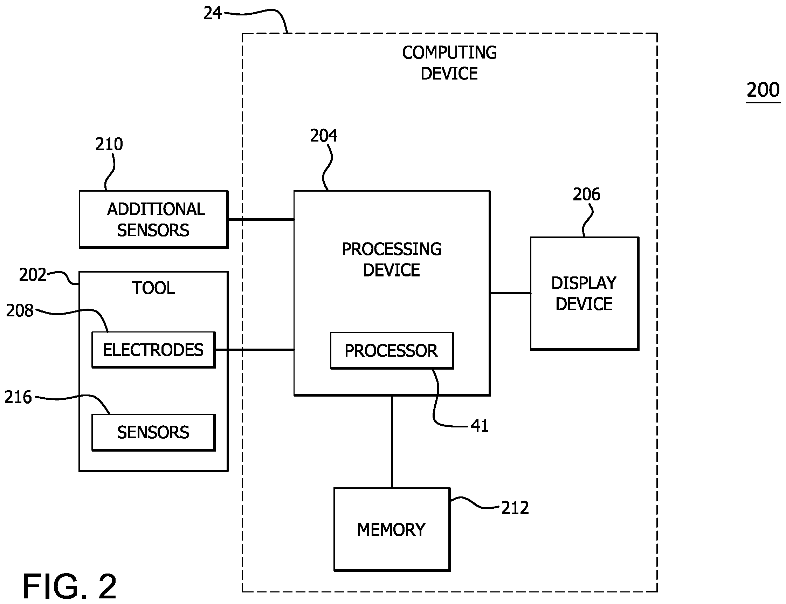

FIG. 2 is a block diagram illustrating example components of a medical system 200 for use with embodiments described herein. As shown in FIG. 2, the system 200 includes a medical tool 202 (e.g., balloon catheter 40 shown in FIG. 1), computing device 24 (e.g., computing device 24 shown in FIG. 1) a processing device 204 comprising processor 41, a display device 206 and memory 212. For explanation purposes, the medical tool 202 will be described herein as a balloon ablation catheter.

As shown in FIG. 2, the processing device 204, display device 206 and memory 212 are a part of the example computing device 24. In some embodiments, the display device 206 may be separate from computing device 24. Computing device 24 may also include other components, such as I/O interfaces (e.g., interface circuits 44) and a relay 60 shown in FIG. 1.

As shown in FIG. 2, balloon ablation catheter 202 can include one or more sensors 216, which include, for example, a magnetic field location sensor (e.g., sensor 38 in FIG. 1) for providing location signals to indicate the 3D position coordinates of the catheter 202. Sensors 216 also include, for example, position sensors, pressure or force sensors, temperature sensors, impedance sensors or other sensors which provide signals indicating parameters values (e.g., catheter position stability, impedance and TPI) during the medical procedure. In some procedures, one or more additional sensors 210 that are separate from the catheter 202, as shown in example system 200, are also used to provide location signals.

The catheter 202 also includes electrodes 208. The electrodes 208 include, for example, the ablation electrodes 50, the stem electrode 51 and the edge electrode 52 shown in FIG. 1. Catheter 202 is configured to be navigated within the patient anatomy, during a medical procedure, such that the electrodes 208 become in contact with or in close proximity to the tissue (e.g., heart tissue). Ablation electrodes 50 are configured to apply RF energy and ablate tissue in patient anatomy.

Memory 212 includes non-volatile memory, such as random access memory (RAM), dynamic RAM, or a cache. Memory 212 also includes, for example, storage, such as, fixed storage (e.g., a hard disk drive and a solid state drive) and removable storage (e.g., an optical disk and a flash drive).

Display device 206 is configured to display one or more maps of the heart in the 3D space including data corresponding to the acquired electrical signals (i.e., electrical signal data) of the portion of patient anatomy (e.g., the heart). For example, display device 206 is configured to display maps representing a spatio-temporal manifestation of the heart as well as the electrical signal data at regions of the heart on the maps. Display device 206 may be in wired or wireless communication with processing device 204. In some embodiments, display device may be separate from computing device 24. Display device 206 may include one or more displays each configured to display one or more maps.

Processor 41 is configured to perform measurements during an ablation procedure, which include measuring one or more first impedances between one or more of the ablation electrodes and the stem electrode, measuring one or more second impedances between one or more of the ablation electrodes and the edge electrode and, based on the first and second impedances, determine, for at least an ablation electrode from among the one or more ablation electrodes, whether the ablation electrode is in physical contact with the wall tissue.

Processor 41 is also configured to perform additional impedance calculations and measurements, as part of a lesion assessment, the results of which enable a physician to predict whether or not a lesion was formed by the ablation.

Processor 41 also configured to processes the acquired impedance calculations and measurements signals (e.g., from electrodes 208) as electrical signal data and store, in memory 212, the electrical signal data acquired via electrodes 208. Processing device 204 is also configured to filter the acquired electrical signal data according to one or more filter parameter settings, generate mapping information and drive display device 206 to display the maps using the mapping information.

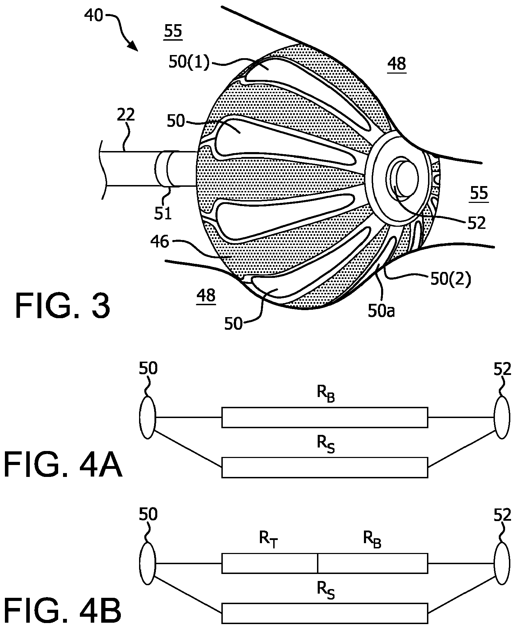

FIG. 3 is an illustration of the example balloon catheter 40 shown in FIG. 1 in physical contact with cavity wall tissue 48. Balloon catheter 40 comprises a plurality of ablation electrodes 50 which are disposed over a membrane 46 of the balloon. Stem electrode 51 and edge electrode 52 are disposed on the distal end of shaft 22 and are immersed in blood 55. In the example shown in FIG. 3, the ablation electrode 50(1) disposed at the top of membrane 46 is in full contact with the tissue 48 (i.e., the tissue 48 is in contact with the full contact area of the electrode 50). In contrast, electrode 50(2) disposed at the bottom of membrane 46 has a distal area 50a that is immersed in blood 55. Accordingly, different impedance values are acquired between the top and bottom ablation electrodes (50(1) and 50(2)) and the edge electrode 52, which are indicative of the full contact and partial contact, respectively, with tissue. The balloon catheter 40 also includes additional elements (e.g., temperature sensors and irrigation holes) which are omitted from the illustration shown in FIG. 3 for simplified explanation and clarity of features of the present disclosure.

FIG. 4A is a schematic diagram of an ablation electrode 50 electrically coupled to the edge electrode 52 shown in FIG. 3 while the ablation electrode 50 is in partial contact with the tissue 48. FIG. 4B is a schematic diagram of an ablation electrode 50 electrically coupled to the edge electrode 52 shown in FIG. 3 while the ablation electrode 50 is in full contact with the tissue 48.

The diagram in FIG. 4A illustrates an example of ablation electrode 50 having a distal area, such as area 50a shown in FIG. 3, that is immersed in blood 55, resulting in electrode 50 having insufficient tissue contact. The impedance between ablation electrode 50 and edge electrode 52 equals the blood impedance, $R_B$, in parallel to a shunt resistance Rs, which can result from blood, tissue and/or another electrically conductive intra-body channel. The insufficient impedance is represented as shown below in Equation 1:

$$| Z\_insufficient | = R_B \| R_S \qquad\qquad \text{Equation 1}$$

For the case in which a balloon is mostly immersed in blood such that the shunt resistivity is dominated by blood resistivity, a minimum value of Z_insufficient is about $R_B/2$. For the case in which the shunt resistivity is infinite, a maximum value of Z_insufficient is $R_B$. For typical blood resistivity value of approximately 100 Ohms, Z_insufficient falls in the range of approximately 50 to approximately 100 Ohms.

The diagram in FIG. 4B illustrates an example of an ablation electrode 50 which is in full contact with tissue. As shown, the impedance between ablation electrode 50 and edge electrode 52 is of blood in series with tissue, $R_B + R_T$, in parallel to the shunt resistance Rs. This sufficient impedance is represented as shown below in Equation 2:

$$| Z\_sufficient | = ( R_B + R_T ) \| R_S \qquad\qquad \text{Equation 2}$$

Because tissue impedance is considerably larger than blood impedance, a "sufficient" impedance is typically larger than an "insufficient" impedance by a value large enough to be measured (e.g., at least several ohms) and, therefore, a case in which an ablation electrode 50 is in partial contact with tissue (i.e., immersed in blood) can be distinguished from a case in which the ablation electrode 50 is full contact with the tissue 48, using, for example, a calibrated threshold impedance value.

For the case in which a balloon is mostly immersed in blood such that the shunt resistivity is dominated by blood resistivity, a minimum value of Z_sufficient is about $R_B$. In this case, the balloon is repositioned due to low shunt resistivity. A practical threshold value for Z_sufficient is $R_T$ to account for the case in which a shunt resistivity is mainly via tissue. For a typical blood resistivity value of approximately 100 Ohms and a tissue resistivity value of approximately 300 Ohms, Z_sufficient is above approximately 150 Ohms. A lower number above approximately 100 ohms, can be used, however, as a threshold for Z_sufficient depending, for example, on measurement repeatability.

Although FIGS. 4A and 4B are described above with regard to the impedance between the ablation electrode 50 and the edge electrode 52, a case of partial contact with tissue can also be distinguished from a case of full contact with the tissue by measuring the impedance between the ablation electrode 50 and the stem electrode 51.

Processor 41 determines, for example, that the ablation electrode is in physical contact with the tissue by determining that a measured first or second impedance is equal to or greater than a predetermined impedance value (e.g., a threshold impedance value) stored in a look-up table. An example of a look-up table which includes levels of contact and corresponding threshold impedance values is shown below in Table 1.

Table 1

| Level of Contact | Threshold Impedance Value |
|---|---|
| Minimally sufficient | About 110 Ohms |
| Sufficient | About 130 Ohms |
| Good | About 150 Ohms |
| Excellent | About 200 Ohms |

By measuring the impedance between ablation electrodes 50 and both stem electrode 51 and edge electrode 52, full physical contact with tissue from both ends of the elongated ablation electrodes can be confirmed. The levels and the number of levels shown in Table 1 are merely an example. Features of the disclosure can be implemented using different levels and different numbers of levels than those shown in Table 1.

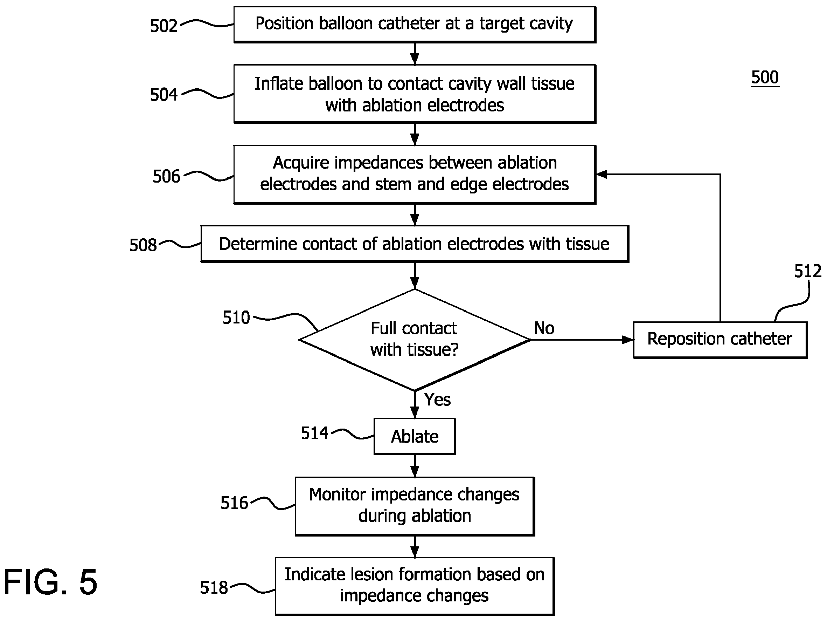

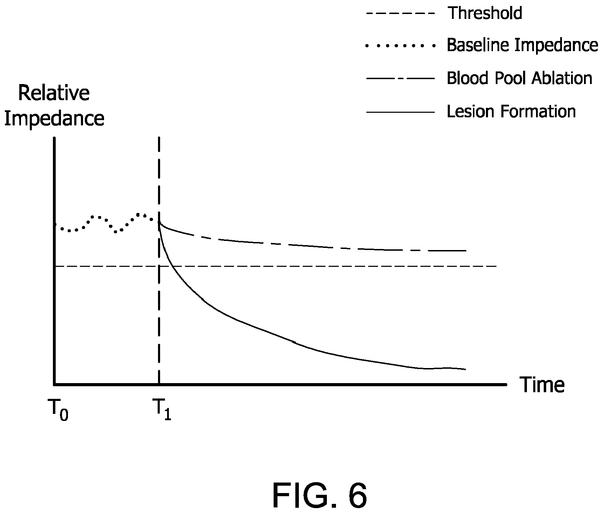

FIG. 5 is a flow chart illustrating a method 500 of lesion assessment formation according to features of the present disclosure. The method which is shown in FIG. 5 and described below determines whether a lesion is formed from ablation by monitoring the changes in impedance characteristics during ablation. The changes in impedance characteristics are provided (e.g., displayed to a physician) as an indication that a lesion is formed. That is, from the indication, the physician can predict whether a lesion is formed. Examples of different types of visually displayed impedance information is shown in FIG. 6, which is described in more detail below.

As shown at block 502, a partially expanded balloon catheter (e.g., catheter 40) is positioned (e.g., by physician 30) at a target location inside a cavity of patient anatomy (e.g., an ostium of a pulmonary vein of a heart). As shown at block 504, the balloon is expanded to bring an ablation electrode (e.g., an ablation electrode 50) into full contact with tissue.

As shown at block 506, the method 500 includes acquiring impedance measurements between each of the ablation electrodes 50 (i.e., balloon electrodes) and the stem electrode 51 and impedance measurements between each of the ablation electrodes 50 and the edge electrode 52. For example, for each one of the ablation electrodes 50, the impedance Z1 between the edge electrode 52 and an ablation electrode 50 can be represented as shown in Equation 3 below:

$$Z1 = Z_{\text{Edge}} - Z_{\text{Balloon}} \qquad \text{Equation 3}$$

For each one of the ablation electrodes 50, the impedance Z2 between the stem electrode 51 and an ablation electrode 50 can be represented as shown in Equation 4 below:

$$Z2 = Z_{\text{Edge}} - Z_{\text{Balloon}} \qquad \text{Equation 4}$$

As shown at block 508, based on the acquired impedance measurements a level of contact sufficiency (e.g., full or partial contact) between each of the ablation electrodes and the tissue is determined (e.g., via processor 41). For example, processor 41 determines a level of contact for each ablation electrode 50 by comparing a measured unipolar impedance value to one or more stored unipolar threshold impedance values, such as those shown in Table 1 above. The levels of contact can be indicated (e.g., displayed) to a physician for identifying whether the electrode is in full contact with tissue. For example, a physician can determine that the electrode is in full contact from any of the displayed levels of contact (e.g., minimally sufficient, sufficient, good and excellent shown in Table 1.

Additionally or alternatively, a level of contact by each ablation electrode with the tissue can be determined based on a relative bipolar impedance RI, which can be represented as shown in Equation 5 below:

$$\text{RI} = \frac{Z1 - Z2}{Z1 + Z2} \qquad \text{Equation 5}$$

When there is no contact between an ablation electrode 50 and the tissue, both impedance measurements (Z1 and Z2) will be approximately equal to each other and, therefore, the relative bipolar impedance RI is equal to or approximately equal to zero and can be represented as shown in Equation 6 below:

$$RI(no\ contact) = \frac{Z1 - Z2}{Z1 + Z2} = \frac{Z_{\text{blood}} - Z_{\text{blood}}}{Z_{\text{blood}} + Z_{\text{blood}}} \approx 0 \qquad \text{Equation 6}$$

When there is contact between an ablation electrode 50 and the tissue, the impedance measurements (Z1 and Z2) will be different from each other and, therefore, the relative bipolar impedance RI is not equal to zero and can be represented as shown in Equation 7 below:

$$RI(contact) = \frac{Z1 - Z2}{Z1 + Z2} = \frac{Z_{\text{Tissue}} - Z_{\text{Blood}}}{Z_{\text{Tissue}} + Z_{\text{Blood}}} \neq 0 \qquad \text{Equation 7}$$

Accordingly, when there is contact between an ablation electrode 50 and tissue, the relative bipolar impedance has a value which is not equal to zero. When there is contact between an ablation electrode 50 and tissue, the relative bipolar impedance is equal to zero.

When it is determined, at decision block 510, that each of the ablation electrodes 50 are not in full contact with tissue (NO decision) due, for example, to insufficient impedance (Table 1) as measured by the electrodes, the catheter 40 is repositioned to improve contact, and the method proceeds back to block 506 to reassess a level of contact sufficiency between each of the ablation electrodes 50 and the tissue. When it is determined, at decision block 510, that each of the ablation electrodes 50 are in full contact with tissue (YES decision), ablation of the tissue begins at block 514.

Ablation of the tissue is performed by applying, for example, RF energy by one or more of the ablation electrodes to a region of the tissue 48. During the ablation procedure, changes to the impedance characteristics (e.g., changes to unipolar impedance values and changes to relative bipolar impedance values) are monitored, at block 516, and indications of the impedance changes are provided (e.g., displayed), at block 518, to a physician. The indicated changes can be used by the physician to predict whether a lesion is successfully formed on the tissue 48. The impedance measurements described herein correspond to an alternating current (AC) applied in an RF range of about 10 kHz to about 500 kHz.

The impedance of tissue is typically higher than the impedance of blood. When a lesion is formed by ablation of the tissue, however, the electrical characteristics of the tissue change and the impedance, at the region of the tissue 48 where the lesion is formed, is reduced (e.g., about 15-20 ohms). Accordingly, an impedance change of about 15 to about 20 ohms is an indication that a lesion is successfully formed at the target site of the tissue 48.

For example, processor 41 monitors changes between previously acquired unipolar impedance values (e.g., values between each of the ablation electrodes 50 and the edge electrode 52) and unipolar impedance values acquired during ablation and the changes between the unipolar impedance values are provided (e.g., displayed) to a physician. In some cases, the previously acquired unipolar impedance values are acquired prior to ablation. Alternatively, the previously acquired unipolar impedance values are also acquired during ablation.

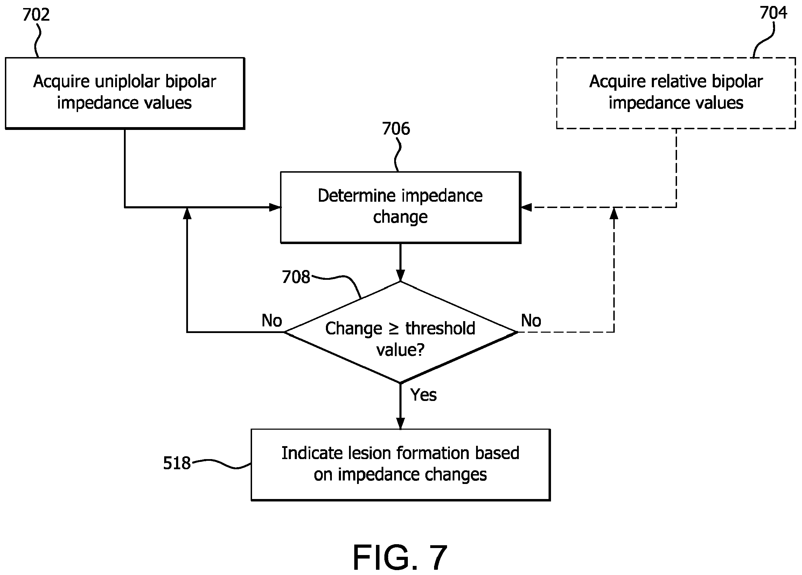

FIG. 7 is a flow chart illustrating an example method 700 of indicating lesion formation based on impedance changes according to features of the present disclosure. As shown at block 702, in one embodiment, the acquired impedance values are unipolar impedance values. For example, the processor 41 stores the unipolar impedance values previously acquired during ablation, (e.g., first unipolar impedance values) and determines, for each electrode, a change in impedance, at block 706, by comparing the stored unipolar impedance values to subsequently acquired unipolar impedance values (e.g., second unipolar impedance values) acquired prior to ablation or during ablation (e.g., by the monitoring at block 516 in FIG. 5). The change between the unipolar impedance values is compared to a threshold value (e.g., unipolar impedance threshold value) and when a change between the unipolar impedance values is determined, at decision block 708, to be equal to or greater than the threshold value (e.g., a value in the range of approximately 15 to approximately 20 ohms), an indication of lesion formation (e.g., that the impedance change is equal to or greater than the threshold value) is provided to the physician, at block 518 (shown in both FIG. 5 and FIG. 7), which can be used to predict the formation of a lesion. When the change between the unipolar impedance values is determined, at decision block 708, to be less than the threshold value, the method proceeds back to block 706 to monitor impedance changes.

Additionally or alternatively, processor 41 monitors changes to the relative bipolar impedance values described above. For example, as shown in by the dashed lines at block 704, the acquired impedance values are bipolar impedance values. For example, because the impedance at a region of tissue drops by about 15-20 ohms when a lesion is formed, the relative bipolar impedance values (shown above in Equation 5) also changes. When the ablation is started, the processor 41 can store the relative bipolar impedance values and then compare relative bipolar impedance values acquired during ablation to the stored relative bipolar impedance values. Similar to the unipolar impedance values described above, the processor 41 determines, for each electrode, a change in impedance at block 706, by comparing the stored bipolar impedance values to subsequently acquired relative bipolar impedance values (e.g., second unipolar impedance values) acquired prior to ablation or during ablation (e.g., by the monitoring at block 516 in FIG. 5). When the change between a relative bipolar impedance value is determined, at decision block 708, to be equal to or greater than a threshold value (e.g., bipolar impedance threshold value), an indication of lesion formation (e.g., that the impedance change is equal to or greater than the threshold value) is provided to the physician, at block 518 (shown in FIG. 5 and FIG. 7), which can be used to predict the formation of a lesion. When the change between the bipolar impedance values is determined, at decision block 708, to be less than the threshold value, the method proceeds back to block 706 to monitor impedance changes.

The displayed changes to impedance values can include different types of visually displayed information so that a physician can predict that a lesion is successfully formed.

FIG. 6 illustrates examples of different types of visually displayed impedance information, acquired over time during a medical procedure, which can be used by a physician for predicting whether a lesion is formed. The impedance measurement information shown in FIG. 6 includes baseline relative impedance information, relative impedance information indicating a blood pool ablation, relative impedance information indicating lesion formation and a threshold. The X axis represents time and the Y axis represents bipolar relative impedance values between an ablation electrode 50 and an edge electrode 52. The information shown in FIG. 6 can also be de displayed for each of the other ablation electrodes 50.

A baseline relative impedance is acquired between time t0 and time t1 prior to the start of the ablation procedure at time t1. The baseline relative impedance shown in FIG. 6 indicates full contact between an ablation electrode 50 and tissue 48. A portion of tissue is ablated, at time t1, by one or more of the ablation electrodes 50 of the balloon catheter shown in FIG. 3.

The horizontal dashed line shown in FIG. 6 represents a threshold value. The threshold value is, for example, determined based on a baseline relative impedance at time t1 and the impedance drop of about 15-20 ohms resulting from lesion formation as described above. The top curved line shown in FIG. 6 represents a blood pool ablation. That is, when there is little or no change between the baseline relative impedance and the relative impedance during the ablation, there is a visual indication of a blood pool ablation. The bottom curved line shown in FIG. 6 represents a lesion formation. That is, when there is significant change (change greater than a threshold value) between the baseline relative impedance and the relative impedance during the ablation, there is a visual indication of lesion formation.

The methods provided can be implemented in a general purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer readable media). The results of such processing can be maskworks that are then used in a semiconductor manufacturing process to manufacture a processor which implements features of the disclosure.

The methods or flow charts provided herein can be implemented in a computer program, software, or firmware incorporated in a non-transitory computer-readable storage medium for execution by a general purpose computer or a processor. Examples of non-transitory computer-readable storage mediums include a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. Although features and elements are described above in particular combinations, each feature or element can be used alone without the other features and elements or in various combinations with or without other features and elements.

Aspects of the invention:

1. A method of lesion assessment for a medical ablation procedure, the method comprising:

acquiring first impedance measurements between each of a plurality of ablation electrodes of a medical probe and a stem electrode of the medical probe;
acquiring second impedance measurements between each of the ablation electrodes of the medical probe and an edge electrode of the medical probe;
ablating tissue of patient anatomy;
determining, during ablation of the tissue, changes to at least one of the first and second impedance measurements; and
indicating lesion formation based on the changes to at least one of the first and second impedance measurements.

2. The method of aspect 1, wherein the indicating of the lesion formation based on the changes to the at least one of the first and second impedance measurements comprises displaying the changes.

3. The method of aspect 1, wherein the medical probe comprises a balloon catheter.

4. The method of aspect 1, wherein the first impedance measurements and the second impedance measurements are acquired prior to ablating the tissue of patient anatomy.

5. The method of aspect 1, wherein determining the changes to the at least one of the first and second impedance measurements comprises continuously monitoring the changes during ablation of the tissue.

6. The method of aspect 1, wherein the changes to the at least one of the first and second impedance measurements are changes to unipolar impedance values.

7. The method of aspect 6, further comprising:

comparing, for each ablation electrode, a change between a first unipolar impedance value and a second unipolar impedance value to a unipolar impedance threshold value; and
indicating the lesion formation based on the change between the first unipolar impedance value and the second unipolar impedance value when the change between the first unipolar impedance value and the second unipolar impedance value is equal to or greater than the unipolar impedance threshold value.

8. The method of aspect 1, wherein the changes to the at least one of the first and second impedance measurements are changes to relative bipolar impedance values.

9. The method of aspect 8, further comprising:

comparing, for each ablation electrode, a change between a first relative bipolar impedance value and a second relative bipolar impedance value to a relative bipolar impedance threshold value; and
indicating the lesion formation based on the change between the first relative bipolar impedance value and the second relative bipolar impedance value when the change between the first relative bipolar impedance value and the second relative bipolar impedance value is equal to or greater than the relative bipolar impedance threshold value.

**Claims**

1. A processing device for use during a medical procedure, the processing device comprising:

memory configured to store data; and
a processor configured to:

acquire first impedance measurements between each of a plurality of ablation electrodes of a medical probe and an edge electrode of the medical probe;
acquire second impedance measurements between each of the ablation electrodes and a stem electrode of the medical probe;
determine, during ablation of tissue of patient anatomy, changes to at least one of the first and second impedance measurements; and
indicate lesion formation based on the changes to at least one of the first and second impedance measurements.

2. The processing device of claim 1, wherein the processor is configured to indicate the lesion formation based on the changes by displaying the changes to the at least one of the first and second impedance measurements on a display device.

3. The processing device of claim 1, wherein the medical probe is a balloon catheter.

4. The processing device of claim 1, wherein the processor is configured to:

acquire the first impedance measurements and the second impedance measurements prior to ablating the tissue of patient anatomy; and
store the first impedance measurements and the second impedance measurements as impedance data in the memory.

5. The processing device of claim 1, wherein the processor is configured to determine the changes to the at least one of the first and second impedance measurements by continuously monitoring the changes during the ablation of the tissue.

6. The processing device of claim 1, wherein the changes to the at least one of the first and second impedance measurements are changes to unipolar impedance values.

7. The processing device of claim 6, wherein the processor is configured to:

compare, for each ablation electrode, a change between a first unipolar impedance value and a second unipolar impedance value to a unipolar impedance threshold value; and

indicate the lesion formation based on the change between the first unipolar impedance value and the second unipolar impedance value when the change between the first unipolar impedance value and the second unipolar impedance value is equal to or greater than the unipolar impedance threshold value.

8. The processing device of claim 1, wherein the changes to the at least one of the first and second impedance measurements are changes to relative bipolar impedance values.

9. The processing device of claim 8, wherein the processor is configured to:

compare, for each ablation electrode, a change between a first relative bipolar impedance value and a second relative bipolar impedance value to a relative bipolar impedance threshold value; and

indicate the change between the first relative bipolar impedance value and the second relative bipolar impedance value when the change between the first relative bipolar impedance value and the second relative bipolar impedance value is equal to or greater than the relative bipolar impedance threshold value.

10. A system for use with performing a medical procedure, the system comprising:

a balloon catheter, comprising a plurality of ablation electrodes configured to ablate tissue of patient anatomy, a stem electrode and an edge electrode;;

a processing device comprising a processor configured to:

acquire first impedance measurements between each of the ablation electrodes of the balloon catheter and an edge electrode of the balloon catheter;

acquire second impedance measurements between each of the ablation electrodes of the balloon catheter and a stem electrode of the balloon catheter;

determine, during ablation of the tissue, changes to at least one of the first and second impedance measurements; and

indicate lesion formation based on the changes to at least one of the first and second impedance measurements.

11. The system of claim 10, further comprising a display device, wherein the processor is configured to indicate the lesion formation based on the changes by displaying the changes to the at least one of the first and second impedance measurements on the display device.

FIG. 1

FIG. 2

EP 4 197 471 A1

FIG. 3

FIG. 4A

FIG. 4B

EP 4 197 471 A1

500

| Block | Description |
|-------|-------------|
| 502 | Position balloon catheter at a target cavity |
| 504 | Inflate balloon to contact cavity wall tissue with ablation electrodes |
| 506 | Acquire impedances between ablation electrodes and stem and edge electrodes |
| 508 | Determine contact of ablation electrodes with tissue |
| 510 | Full contact with tissue? |
| 512 | Reposition catheter |
| 514 | Ablate |
| 516 | Monitor impedance changes during ablation |
| 518 | Indicate lesion formation based on impedance changes |

No — Reposition catheter

Yes — Ablate

FIG. 5

Threshold
Baseline Impedance
Blood Pool Ablation
Lesion Formation

Relative Impedance

$T_0$

$T_1$

Time

FIG. 6

EP 4 197 471 A1

702

Acquire uniplolar bipolar
impedance values

704

Acquire relative bipolar
impedance values

706

Determine impedance
change

708

No ⟵ Change ≥ threshold
value? ⟶ No

Yes

518

Indicate lesion formation based
on impedance changes

FIG. 7

EP 4 197 471 A1

Europäisches
Patentamt

European
Patent Office

Office européen
des brevets

# EUROPEAN SEARCH REPORT

Application Number

EP 22 21 3304

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2019/274581 A1 (MOSESOV ARTEM [US] ET AL) 12 September 2019 (2019-09-12) * abstract; claims 1-14; figures 1-4 * * paragraphs [0004] - [0048] * ----- | 1-11 | INV. A61B18/14 ADD. A61B18/00 |
| Y | US 2017/354449 A1 (AVITALL BOAZ [US] ET AL) 14 December 2017 (2017-12-14) * abstract; claims 1-19; figures 1-26 * * paragraphs [0007] - [0123] * ----- | 1-11 | |
| A | US 2021/059743 A1 (GOVARI ASSAF [IL]) 4 March 2021 (2021-03-04) * abstract; claims 1-20; figures 1-9 * * paragraphs [0010] - [0049] * ----- | 1-11 | |
| A | US 2021/106249 A1 (SCHMIDT MEGAN [US] ET AL) 15 April 2021 (2021-04-15) * abstract; claims 1-20; figures 1-5 * * paragraphs [0005] - [0065] * ----- | 1-11 | |

TECHNICAL FIELDS
SEARCHED (IPC)

A61B

The present search report has been drawn up for all claims

| Place of search | Date of completion of the search | Examiner |
|---|---|---|
| Munich | 12 April 2023 | Mendelevitch, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

17

## ANNEX TO THE EUROPEAN SEARCH REPORT
## ON EUROPEAN PATENT APPLICATION NO.

EP 22 21 3304

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2023

| Patent document cited in search report | | Publication date | Patent family member(s) | | Publication date |
|---|---|---|---|---|---|
| US 2019274581 | A1 | 12-09-2019 | CN | 111479497 A | 31-07-2020 |
| | | | EP | 3668381 A1 | 24-06-2020 |
| | | | JP | 7106644 B2 | 26-07-2022 |
| | | | JP | 2021528108 A | 21-10-2021 |
| | | | US | 2019183378 A1 | 20-06-2019 |
| | | | US | 2019274581 A1 | 12-09-2019 |
| | | | WO | 2019126260 A1 | 27-06-2019 |
| US 2017354449 | A1 | 14-12-2017 | CA | 2931860 A1 | 11-06-2015 |
| | | | CN | 106061421 A | 26-10-2016 |
| | | | EP | 3076888 A1 | 12-10-2016 |
| | | | US | 2015157382 A1 | 11-06-2015 |
| | | | US | 2017354449 A1 | 14-12-2017 |
| | | | US | 2019365452 A1 | 05-12-2019 |
| | | | US | 2021290285 A1 | 23-09-2021 |
| | | | WO | 2015081420 A1 | 11-06-2015 |
| US 2021059743 | A1 | 04-03-2021 | CN | 112438793 A | 05-03-2021 |
| | | | EP | 3785656 A1 | 03-03-2021 |
| | | | IL | 276742 A | 01-03-2021 |
| | | | JP | 2021030086 A | 01-03-2021 |
| | | | US | 2021059743 A1 | 04-03-2021 |
| US 2021106249 | A1 | 15-04-2021 | CN | 114502067 A | 13-05-2022 |
| | | | EP | 4041111 A1 | 17-08-2022 |
| | | | US | 2021106249 A1 | 15-04-2021 |
| | | | WO | 2021071658 A1 | 15-04-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7756576 B
- US 7869865 B
- US 7848787 B
- US 8456182 B